# EUROPEAN PATENT APPLICATION

(11) **EP 1 870 123 A1**
(43) Date of publication of application: **26.12.2007**
(21) Application number: 06732390.7
(22) Date of filing: 13.04.2006
(51) Int. Cl.: A61M 16/10, B01D 53/04

(54) **OXYGEN ENRICHING DEVICE**

(30) Priority: 15.04.2005 JP 2005118060
(71) Applicant: Teijin Pharma Limited, Tokyo 100-0011 (JP)
(72) Inventor: TAKEMASA, Kenji, Teijin Pharma Limited, Iwakuni-shi, Yamaguchi 740-0014 (JP)
(74) Representative: HOFFMANN EITLE
(86) International application number: PCT/JP2006/308784
(87) International publication number: WO 2006/112546

(57) **Abstract**

The present invention provides a pressure swing adsorption-type oxygen concentrator for medical use having a flow channel switching device that successively pressurizes and supplies air from an air supply device at a constant timing to adsorb/desorb nitrogen in the air, wherein the flow channel switching device is a rotary distributor valve assembly structure provided with an electric motor, the rotary distributor valve assembly structure being provided with a shaft seal mechanism to perform shaft sealing with an O-ring having Shore A hardness of 45 to 60 degree and further the oxygen concentrator has a mechanism that simultaneously achieves rotary drive transmission following axial offset and angular offset of the rotary shaft of the rotary distributor valve assembly structure and shaft sealing.

## Description

### Technical Field

The present invention relates to an oxygen concentrator that separates an oxygen-concentrated gas containing oxygen-enriched air from air. More particularly, the present invention relates to a shaft seal mechanism in flow channel switching device that has a rotary distributor valve assembly structure to successively pressurize and supply air to adsorption beds at a constant timing to absorb/desorb nitrogen in the air in an adsorption-type oxygen concentrator having a plurality of adsorption beds.

### Background Art

Recently, the number of patients suffering from respiratory diseases such as asthma, pulmonary emphysema, chronic bronchitis and the like has tended to increase and one of the most effective treatment methods for these diseases is oxygen inhalation therapy. As a supply source of oxygen used in such therapy, an oxygen concentrator that directly separates an oxygen-enriched gas from air has been developed. The concentrator has become gradually popular as a therapeutic device for oxygen inhalation therapy because of its convenience in use, easiness in maintenance management and the like.

As one of such oxygen concentrators, a multiple-cylinder type of adsorption-type oxygen concentrator equipped with one, two, or more adsorption cylinders each housing one or more adsorption beds filled with an adsorbent capable of selectively adsorbing nitrogen or oxygen is known. A pressure swing adsorption (PSA)-type device using a pressure range from increased pressure to ordinary pressure and a vacuum pressure swing adsorption (VPSA)-type device using a pressure range from increased pressure to vacuum are used, both of which use a compressor as a supplier of raw material air.

By bringing one or more absorption beds filled with an adsorbent capable of selectively adsorbing nitrogen to a pressurized condition by supplying compressed air using a compressor, an oxygen-concentrated gas can be obtained by successively conducting at a certain cycle of the following steps: an adsorption step in which nitrogen is adsorbed on an adsorbent to obtain an unadsorbed oxygen-concentrated gas; a desorption step in which an inner pressure of the absorption beds is reduced to desorb nitrogen and to perform regeneration of the adsorbent; and further, immediately before the completion of the desorption step, a pressure equalizing step in which part of a dry oxygen-concentrated gas already produced is inversely flown to improve regeneration efficiency and to increase pressure.

Such an adsorption-type oxygen concentrator generally has such a flow that a compressor is connected to an adsorption bed via tubing, and a switching valve is placed between the compressor and the adsorption bed to switch a supply route of pressurized air to the adsorption bed. The concentrator has, however, problems that the number, the type and the like of parts such as switching valves increase due to the number of adsorption beds and the conduct of complicated control of adsorption/desorption steps, and troubles in maintenance occur. According to Japanese Patent Application Laid-Open No. H07-508205, an oxygen concentrator equipped with a fluid separator in which all oxygen concentration function parts such as a plurality of adsorption beds, connecting flow channels and a rotary valve for switching flow channels and the like are made into a module is developed, as a device to overcome such troubles.

### Disclosure of the Invention

In order to use such an adsorption-type oxygen concentrator at home or for medical use, it is essential to reduce a size and a cost of the device. Integration of oxygen generation devices including a rotary valve as a device to switch plural flow channels as a module is a potent measure to reduce the size of the device and the number of parts. As such an oxygen generation module, Japanese Patent Application Laid-Open No. H07-508205 discloses a fluid separator provided with a plurality of adsorption cylinders, in which gas supply channels from a compressor to the absorption cylinders, connecting flow channels between the adsorption cylinders, and an exhaust flow channel are made into a manifold and the flow channels are switched by one rotary valve. The flow channel switching device used in this device is provided with a mechanically sealed rotary valve and a fixed valve part integrated with a flow channel manifold. If size reduction of the module is desired, it is difficult to provide a sufficient mechanism space in the valve. In addition, since its structure is such that load is applied in an axial direction against a shaft center due to the mechanism of the mechanical seal, a thin thrust bearing is often used as a bearing of a rotary valve. However, the thin thrust bearing is difficult to bear load in a radial direction, and as a result, alignment of the rotary shaft tends to suffer from axial offset in this structure. Further, commercially available thrust bearings have no mechanism to seal grease in their structures even if grease is contained, and it is thus known that excessive grease flows out to the outside of the thrust bearings and contaminates the mechanical seal mechanism part to cause troubles.

Further, there exists such a problem that the rotary shaft of the rotary valve must also function as a shaft seal mechanism for a gas over ranges from a low pressure to a high pressure and a low temperature to a high temperature, when such an eccentric load occurs, sufficient shaft seal performance cannot be maintained, or a rotary shaft cannot follow axial offset of alignment, even if shaft seal performance can be maintained, to induce poor rotation.

An object of the present invention is to provide an improved oxygen concentrator comprising a flow channel switching device having a rotary distributor valve assembly structure, which realizes both a rotary drive transmission mechanism that follows both axial offset and angular offset of the rotary shaft of the rotary distributor valve assembly structure and a shaft seal mechanism at the same time.

The present inventors have keenly studied such problems, and as a result, found an oxygen concentrator that can maintain sufficient shaft seal performance even when a shaft seal member is distorted due to axial offset and angular offset in a rotary distributor valve assembly structure in a fluid separator of a conventional oxygen concentrator and can prevent changes in the load of a rotary drive.

In other words, the present invention provides a pressure swing adsorption-type oxygen concentrator for medical use comprising: a plurality of adsorption beds filled with an adsorbent capable of selectively adsorbing nitrogen relative to oxygen; an air supply device that supplies air to the adsorption beds; and a flow channel switching device that successively pressurizes and supplies the air from the air supply device at a constant timing to adsorb/desorb nitrogen in the air, the flow channel switching device being a rotary distributor valve assembly structure provided with an electric motor, the rotary distributor valve assembly structure being provided with a fixed valve that communicates with a manifold constituting connecting flow channels to the plurality of adsorption beds, a rotary valve that switches the connecting flow channels, a rotary drive transmission shaft that transmits rotation movement of the electric motor to the rotary valve, a tolerance at a connecting part between the rotary drive transmission shaft and the rotary valve, and a shaft seal mechanism provided with an O-ring.

Further, the present invention provides the pressure swing adsorption-type oxygen concentrator, comprising: an inlet port for pressurized air from the air supply device on the fixed valve side; and a pressurized air supply port, wherein the pressurized air is supplied from a sliding face side of the rotary valve to a connecting part between the rotary valve and the rotary drive transmission shaft, the connecting part being on the reverse side of the sliding face of the rotary valve.

In addition, the present invention provides the pressure swing adsorption-type oxygen concentrator, wherein the shaft seal mechanism has a sealing mechanism achieved by an outside diameter and an inside diameter of the O-ring by compression of the O-ring and the O-ring constituting the shaft seal mechanism has a Shore A hardness of 45 to 60 degree.

Further, the present invention provides the pressure swing adsorption-type oxygen concentrator, wherein the shaft seal mechanism is constituted by the O-ring provided in a compressed state between a groove formed on the rotary drive transmission shaft and an inner wall of the rotary valve capable of being inserted the rotary drive transmission shaft.

Further, the present invention provides a method of shaft sealing of a rotary valve, wherein a tolerance is provided at a connecting part between a rotary drive transmission shaft that transmits rotation movement of an electric motor to the rotary valve that switches a flow channel of pressurized air and the rotary valve, and shaft sealing is achieved by an outside diameter and an inside diameter of an O-ring that has a Shore A hardness of 45 to 60 degree by compression of the O-ring between the rotary drive transmission shaft and the rotary valve.

### Brief Description of the Drawings

Figure 1 is a schematic diagram of an oxygen concentrator for medical use of the present invention.
Figure 2 is a schematic diagram of a shaft seal mechanism of a rotary distributor valve assembly structure used in the oxygen concentrator according to the present invention.
Figure 3 is an enlarged view of the shaft seal mechanism of the rotary distributor valve assembly structure used in the oxygen concentrator of the present invention.
Figure 4 shows changes in rotation load in an environment of 20°C at the time of designing the shaft seal mechanism in Figure 2.
Figure 5 shows changes in rotation load in an environment of 0°C at the time of designing the shaft seal mechanism in Figure 2.

### Best Mode for Carrying Out the Invention

The oxygen concentrator according to the present invention is a pressure swing adsorption-type oxygen concentrator for medical use comprising a plurality of adsorption beds filled with an adsorbent capable of selectively adsorbing nitrogen relative to oxygen; an air supply device that supplies air to the adsorption beds; and a flow channel switching device that successively pressurizes and supplies the air from the air supply device at a constant timing for adsorption/desorption; wherein the flow channel switching device is a rotary distributor valve assembly structure having an electric motor, and the rotary distributor valve assembly structure is shaft sealed by an O-ring having a Shore A hardness of 45 to 60 degree.

The oxygen concentrator of the present invention may be used effectively for an oxygen concentrator module with which pressurized air can be successively supplied to each of a plurality of absorption beds by switching the pressurized air supply flow channel from a compressor via the plurality of absorption beds and a manifold communicating with each of the absorption beds by using the rotary valve.

The rotary distributor valve assembly structure has a mechanical seal structure constituted by a fixed valve and a rotary valve, and is provided with a rotary drive transmission shaft that transmits rotation of an electric motor to the rotary valve, a spring that presses and seals sliding faces between the fixed valve and the rotary valve and a mechanical seal part composed of an O-ring to shaft seal a gas. A bearing receiving the rotary drive transmission shaft is provided with a thrust bearing and a radial bearing.

The shaft seal mechanism of the rotary drive transmission shaft is constituted by a rubber O-ring and its material is selected depending on a temperature range in which the device is used and a gas component. Preferably, nitrile rubber (NBR), fluororubber (FKM), styrene-butadiene rubber (SBR), ethylene-propylene rubber (EPM), ethylene-propylene-diene rubber (EPDM), silicone rubber (VMQ) and the like are used.

With the shaft seal mechanism, sealing of the sliding faces between the fixed valve and the rotary valve is secured and tolerance of machining accuracy of the device is absorbed, and axial offset and angular offset of the rotary shaft are absorbed to absorb variations of torque. Accordingly, it is necessary to secure both functions, flexibility and shaft sealing, between the rotary valve and the rotary drive transmission shaft.

In order to absorb axial offset and angular offset of the rotary drive transmission shaft and to suppress changes in load of the rotary valve, a method in which the rotary drive transmission shaft and the rotary valve are shaft sealed via the O-ring is adopted. As a conventional technique, a floating seal method (see Parker O-Ring Handbook, p.5-19, §5.25; published by Parker Hannifin Corporation USA; http://www.parker.com/o-ring/Literature/05-5700.pdf.) may be used for design. In the floating seal method, shaft sealing is designed so that the O-ring floats with respect to the shaft. It is a structure in which a size and shape are such that shaft sealing is not established when pressure is not applied, and, when pressure is applied, a difference in pressure is utilized to move the O-ring to the seal face in the O-ring groove to shaft seal on the outer diameter face and one of the upper and lower faces.

However, under a low pressure condition and/or a low temperature condition, malfunction occurs such that the O-ring does not move so that shaft seal does not function. In the present invention, the O-ring is provided in a compressed state between a groove provided on the rotary drive transmission shaft and the inner wall of the rotary valve into which the rotary drive transmission shaft can be inserted to achieve shaft sealing by the inner diameter and outer diameter of the O-ring.

When the O-ring is placed in a compressed state, flexibility of the rotary drive transmission shaft decreases to cause variations in torque of the rotary valve following axial offset and angular offset of the rotary shaft. On the other hand, by sealing with the use of shaft seal materials for the O-ring having a rubber hardness (Shore A hardness) of 45 to 60 degree, especially preferably 50 degree, the axial offset and angular offset of the rotary shaft are absorbed and the changes in load are reduced. In addition, rubber material for the O-ring is preferably selected in view of environmental conditions for use and safety. For oxygen concentrators, ethylene propylene diene rubber (EPDM) is preferable especially in terms of heat resistance, safety, and grease resistance.

### Example

Specific examples of the oxygen concentrator for medical use of the present invention will be illustrated below referring to the drawings, as required.

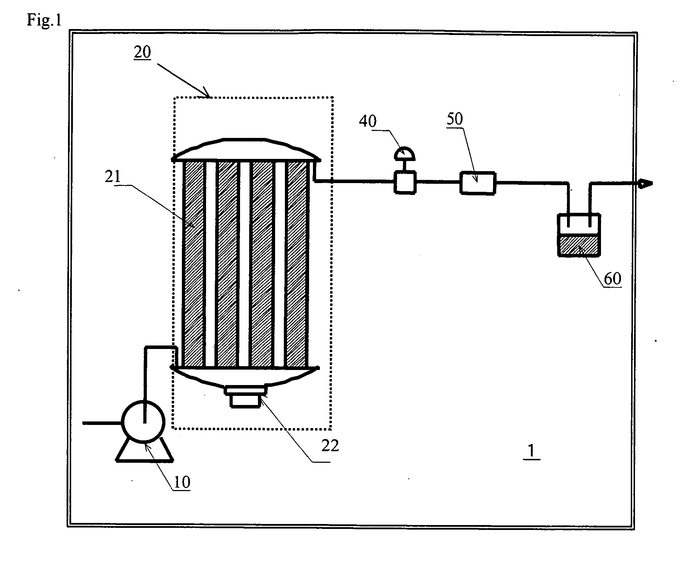

An oxygen concentrator 1 of the present invention for medical use, as shown in Figure 1, is a device that uses air as a raw material; separates and concentrates oxygen in the air by a pressure swing adsorption method; supplies moistured oxygen-concentrated gas to a user; and comprises a compressor 10 as an air supply device, a PSA-type multiple-cylinder adsorption unit 20 (hereinafter sometimes also refer to as ATF module) incorporating a plurality of adsorption beds 21 and a flow channel switching device 22 that supplies pressurized air successively at a constant timing to the adsorption beds to repeat adsorption/desorption, a pressure control valve 40 that controls the produced oxygen-concentrated gas to a predetermined pressure, a flow controller 50, and a moisturizer 60.

In Example of the oxygen concentrator of the present invention, an ATF module (manufactured by TS Precision Co., Ltd.) was used as the multiple-cylinder adsorption unit 20. This ATF module can successively supply pressurized air to the adsorption beds with a rotary valve through 12 adsorption towers and a manifold communicating the respective adsorption cylinders, wherein the structure of the rotary valve itself is a mechanical seal structure constituted by a fixed valve and a rotary valve.

The respective adsorption cylinders were filled with Li-X type molecular sieve zeolite as an adsorbent that can selectively adsorb nitrogen relative to oxygen.

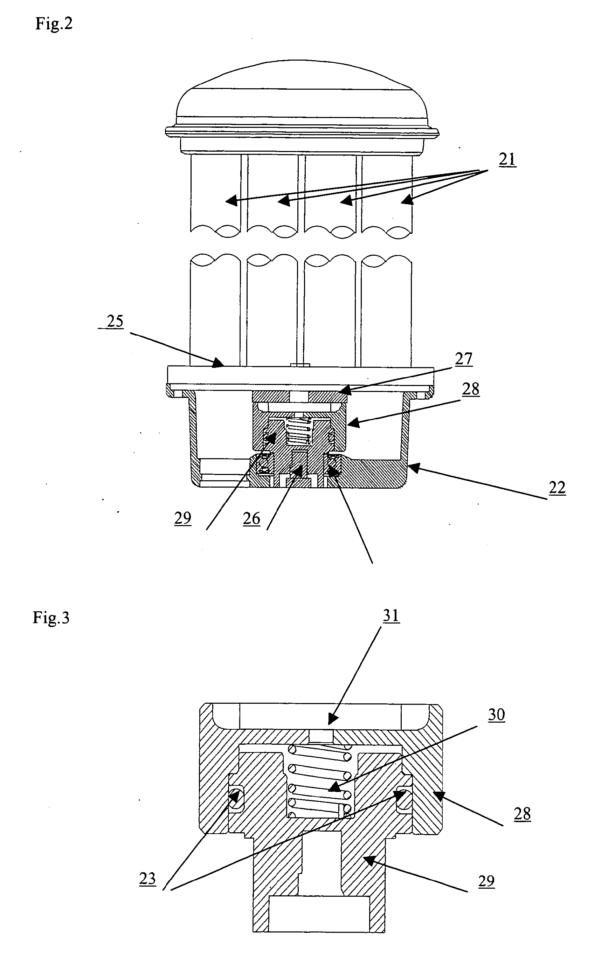

Figures 2 and 3 show schematic constitutions of the rotational valve part of the ATF module. The structure of the flow channel switching device of the rotary distributor valve assembly structure to be incorporated in the ATF module is provided with a fixed valve 27, a rotary valve 28, a rotary drive transmission shaft 29, a spring 30 and a mechanical seal part composed of an O-ring 23 to shaft seal the gas.

In the rotary distributor valve assembly structure, connecting ports to the adsorption cylinder provided concentrically on the fixed valve are connected to flow channels via a switching groove provided on the sliding face of the rotary valve through an inlet port for pressurized air provided to the center from the fixed valve side. As a result, an adsorption step is performed, in which pressurized air is sent to the adsorption cylinders where nitrogen is adsorbed and oxygen is generated. The adsorption cylinders that have completed the adsorption step are connected to the adsorption cylinders that have completed the desorption step via arch-like grooves to equalize the pressure between the adsorption cylinders. A nitrogen-enriched gas is exhausted under reduced pressure from the pressurized adsorption cylinders via the exhaust ports.

A hole 31 to the back face of the sliding face of the fixed valve is provided at the center of the fixed valve. The pressurized air is supplied to the rotary valve to pressurize the sliding face, whereby a force applied to the adsorption port (force pushing up the seal face) is compensated with a force pushing down the seal face that is generated by pressure supplied to the opposite side of the sliding face through the hole 31 placed on the center of the fixed valve. The presence of this central hole determines the force applied on the seal face according to the ratio between the area of the adsorption port and the area of the opposite side. If this hole is not present, on the contrary, as the pressure increases, the force applied to the adsorption port (force pushing up the seal face) increases to deteriorate sealing. In order to compensate the deterioration of the sealing, it is required to hold the seal face with a strong spring to increase a surface pressure. It results in an increase in the sliding torque, and thus an increase in the output of the drive motor is required.

Rotation of the motor rotary shaft 26 rotates the rotary drive transmission shaft 29 and the rotary valve 28 rotates following the rotation of the transmission shaft 29. The O-ring 23 serves as a shaft sealing function between the rotary valve 28 and the rotary drive transmission shaft 29. A spring 30 seals the sliding faces between the rotary valve 28 and the fixed valve 27 by its spring pressure.

Although the sliding faces of the rotary valve 28 and the fixed valve 27 are machined accurately, tolerance is required in a connecting part between the rotary drive transmission shaft 29 and the rotary valve 28 with consideration into machining accuracy and assembly accuracy of the device. Even when axial offset and angular offset of the rotary drive transmission shaft 29 of the rotary valve 28 occur and the O-ring 23 deforms, it is necessary to always maintain the shaft sealing function and to absorb the axial offset and angular offset and suppress changes in rotation load.

A sealing material having Shore A hardness of 70 or more is usually used for the O-ring to maintain sealing performance and abrasion resistance. In this example, a material having hardness (Shore A hardness) of 60 to 45 degree manufactured by EPDM was used for the O-ring 23.

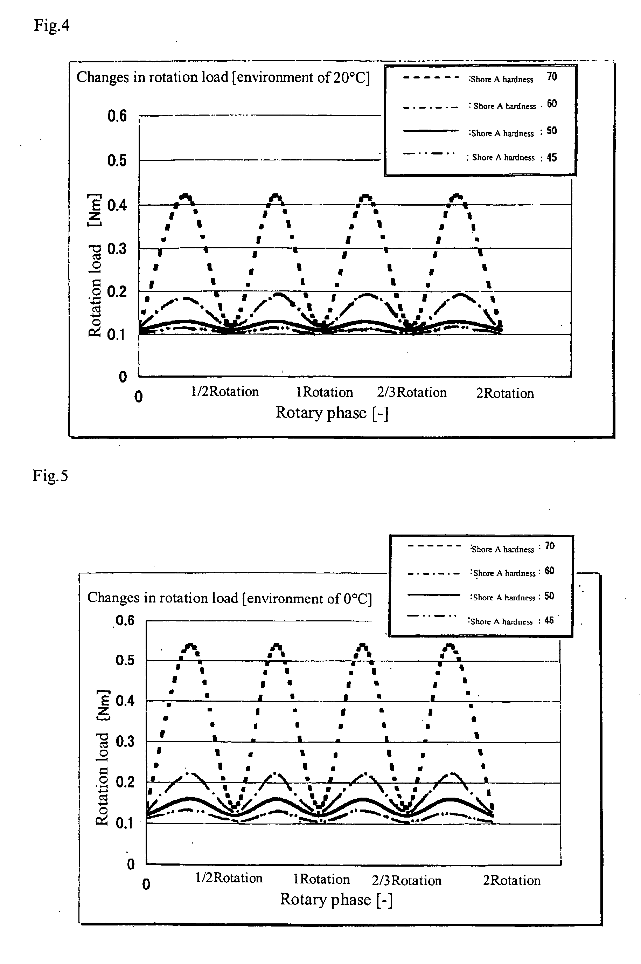

Figures 4 and 5 show the differences in changes in rotation load, by using the shaft seal mechanism shown in Figure 2, between the case where an O-ring made of a hardness rubber in general use (Shore A hardness: 70) is used and the case where O-rings made of a hardness rubber of the invention of the present application (Shore A hardness: 60, 50 and 45) is used. The rubber material was EPDM for all of the O-rings. According to the results shown in Figure 4, the gas was shaft sealed by the shaft seal mechanism using the O-ring having Shore A hardness of 70 degree in an environment at 20°C, but the rotation load changed synchronously to the rotary phase of the rotary shaft. With the O-rings of the invention of the present application having rubber hardness (Shore A hardness) of 60 to 45 degree, shaft sealing of the gas and a decrease in changes in rotation load was achieved. Especially, as Shore A hardness decreased from 60 to 50 and to 45, the changes in rotation load decreased. When Shore A hardness was 45 or less, the material was excessively soft, causing problems such as a problem in durability, difficulties in manufacturing, and easiness in use.

Further, according to the results shown in Figure 5, in an environment at 0°C, rubber hardness tended to increase and it was confirmed that, when the O-ring having Shore A hardness of 70 degree was used, the O-ring 23 was not able to absorb axial offset and angular offset of the rotary drive transmission shaft 29 and changes in rotation load further increased, while the O-rings having rubber hardness corresponding to Shore A hardness of 60 or 45 degree of the invention of the present application, the effect of suppressing the changes in rotation load was retained.

Considering the shaft seal function of the O-ring, effects such as a function to absorb changes in load, durability, manufacturing cost, and the like, a material having Shore A hardness of 50 is the most excellent.

By using the oxygen concentrator of the invention of the present application, an oxygen concentrator that can shaft seal a gas without developing the changes in rotation load due to the generation of axial offset and angular offset loads of the rotary distributor valve assembly structure in the fluid separator can be provided.

## Claims

1. A pressure swing adsorption-type oxygen concentrator for medical use comprising:
a plurality of adsorption beds filled with an adsorbent capable of selectively adsorbing nitrogen relative to oxygen;
an air supply device that supplies air to the adsorption beds; and
a flow channel switching device that successively pressurizes and supplies the air from the air supply device at a constant timing to adsorb/desorb nitrogen in the air,
the flow channel switching device being a rotary distributor valve assembly structure provided with an electric motor,
the rotary distributor valve assembly structure being provided with
a fixed valve that communicates with a manifold constituting connecting flow channels to the plurality of adsorption beds,
a rotary valve that switches the connecting flow channels,
a rotary drive transmission shaft that transmits rotation movement of the electric motor to the rotary valve,
a tolerance at a connecting part between the rotary drive transmission shaft and the rotary valve, and
a shaft seal mechanism provided with an O-ring.

2. The pressure swing adsorption-type oxygen concentrator according to claim 1, comprising:
an inlet port for pressurized air from the air supply device on the fixed valve side; and
a pressurized air supply port, wherein the pressurized air is supplied from a sliding face side of the rotary valve to a connecting part between the rotary valve and the rotary drive transmission shaft, the connecting part being on the reverse side of the sliding face of the rotary valve.

3. The pressure swing adsorption-type oxygen concentrator according to claim 1, wherein the shaft seal mechanism has a sealing mechanism achieved by an outside diameter and an inside diameter of the O-ring by compression of the O-ring and the O-ring constituting the shaft seal mechanism has a Shore A hardness of 45 to 60 degree.

4. The pressure swing adsorption-type oxygen concentrator according to claim 3, wherein the shaft seal mechanism is constituted by the O-ring provided in a compressed state between a groove formed on the rotary drive transmission shaft and an inner wall of the rotary valve capable of being inserted the rotary drive transmission shaft.

5. The pressure swing adsorption-type oxygen concentrator according to claim 3, wherein the O-ring is made of an ethylene-propylene-diene rubber.

6. A method of shaft sealing of a rotary valve, wherein a tolerance is provided at a connecting part between a rotary drive transmission shaft that transmits rotation movement of an electric motor to the rotary valve that switches a flow channel of pressurized air and the rotary valve, and shaft sealing is achieved by an outside diameter and an inside diameter of an O-ring that has a Shore A hardness of 45 to 60 degree by compression of the O-ring between the rotary drive transmission shaft and the rotary valve.

7. The method of shaft sealing of a rotary valve according to claim 6, wherein the O-ring is made of an ethylene-propylene-diene rubber.
